# EUROPEAN PATENT APPLICATION

(11) **EP 2 315 011 A1**
(43) Date of publication of application: **27.04.2011**
(21) Application number: 09802969.7
(22) Date of filing: 29.07.2009
(51) Int. Cl.: G01N 27/16, G01N 27/12

(54) **GAS SENSOR**

(30) Priority: 31.07.2008 JP 2008198715
(71) Applicant: Citizen Finetech Miyota Co., Ltd., Nagano 389-0294 (JP)
(72) Inventor: SHIMIZU Yasuo, Kitasaku-gun Nagano 389-0295 (JP); TAKAHASHI Ikuo, Kitasaku-gun Nagano 389-0295 (JP); KOBAYASHI Hiromichi, Kitasaku-gun Nagano 389-0295 (JP)
(74) Representative: Prüfer & Partner GbR European Patent Attorneys
(86) International application number: PCT/JP2009/063462
(87) International publication number: WO 2010/013727

(57) **Abstract**

Disclosed is a gas sensor in which electrode pins including a plurality of electrode pins (71, 72) which are electrically connected to a gas-sensitive element and support the gas-sensitive element are each made to pass through one surface of a mounting base (in the drawing, provided on the lower side of a holder with spring properties (12)) to the other surface (rear surface) thereof and are supported therein. The end portions of the electrode pins are made to project into grooves (4b) through through-holes (4a) in a spacer (4), and a plurality of lead-out leads (51-53) that are parallel to the rear surface of the mounting base and also extend in a direction parallel to a holder board (11) are connected to projecting portions of the electrode pins by connecting portions (51a, 51b, 52a, 53a). A pressing member (6) is placed thereover and is fixed and held on the holder board (11) by the holder (12) which has spring properties.

## Description

### TECHNICAL FIELD

The present invention relates to a gas sensor used in a wide range of applications such as detecting leakage of various kinds of gases and toxic gas, monitoring of exhaust gas and air pollution, monitoring of various processes and so on, and more particularly to a gas sensor accurately detecting carbon monoxide (CO) generated during incomplete combustion in combustion equipment or leakage of hydrogen gas in a fuel-cell vehicle (FCV).

### BACKGROUND TECHNOLOGY

Conventional sensors detecting flammable gas such as hydrogen gas, methane gas or carbon monoxide gas include a catalytic combustion type gas sensor, a semiconductor type gas sensor and so on. Each of these gas sensors incorporates a heat source used for detecting the flammable gas.

The catalytic combustion type gas sensor has, as described in Patent Document 1, a gas-sensitive element (a gas detecting element) constituted of a heater coil including a combustion catalyst as a heat source and outputs a change in resistance value of the heater coil due to catalytic combustion heat of the flammable gas generated on the combustion catalyst as a change in voltage to thereby detect the presence of the flammable gas.

On the other hand, the semiconductor type gas sensor has a gas-sensitive element constituted of a heater coil including a semiconductor layer as a heat source and outputs a change in electric conductivity of the semiconductor layer caused by the absorption phenomenon of the flammable gas in the semiconductor layer as a change in voltage to thereby detect the presence of the flammable gas.

In these existing gas sensors, the heat source for detecting the flammable gas is provided as described above, and a cap that is a gas-permeable cover member composed of metal mesh, metallic sintered body, porous ceramics or the like is equipped in order to stabilize the thermal equilibrium performance and to secure the explosion-proof performance against the flammable gas.

Further, Patent Document 1 also describes a gas detector in which the aforementioned gas detecting element and a compensating element (temperature compensating element) are connected in series and connected in parallel to a series circuit composed of two resistors connected in series to form a Wheatstone bridge circuit in order to compensate the influence by a change in ambient temperature, and a direct-current voltage is applied across the parallel circuit to detect the voltage between the connection point of the gas detecting element and the compensating element and the connection point of the two resistors. As the compensating element in this case, used is an element in which a heater coil having the same electric characteristic as that of the gas detecting element is buried in a heat conducting layer not coated with or not carrying an oxidation catalyst.

On the other hand, in these existing gas sensors, a mounting base is provided which is made of a synthetic resin having no gas permeability. This mounting base supports pairs of electrode pins passing therethrough, each pair electrically connected to both terminals of the above-described gas detecting element or the compensating element and supporting it, and holds the gas detecting element and the compensating element facing each other in a gas-permeable cap.
When the gas detecting element and the compensating element are set in the same casing as described above, a heat shielding plate made of metal or synthetic resin is equipped between both elements in order to prevent heat interference of both elements.

However, the gas-permeable cap in such a gas sensor has a function of protecting the gas detecting element from environmental factors and, at the same time, has a limit in the gas permeability, thus causing a loss of response performance of the sensor. Further, the existing mounting base is not conducive to permeation of a detection target gas into the sensor and thus does not contribute to the response performance of the sensor.

Further, the heat shielding plate in the catalytic combustion type gas sensor is provided for the purpose of mutually insulating the gas detecting element and the compensating element from heat and, at the same time, also blocks the atmospheric environments around both elements inside the sensor, and therefore is not necessarily preferable for the stability of the output voltage with respect to the temperature and humidity characteristics of the gas sensor.

Hence, proposed one is a gas sensor, as described in, for example, Patent Document 2 including the cap, the mounting base and the heat shielding plate as described above, in which all of them are made of ceramics, preferably, porous ceramics to enable the detection target gas to flow into the gas sensor from all directions so that the gas concentration inside the gas sensor quickly coincides with that of the ambient environment, thereby improving the response performance of the gas sensor output.

Further, as the gas sensor detecting CO₂ or NOₓ, a solid electrolyte gas sensor is also in heavy usage as described, for example, in Patent Document 3. The sensor body being the gas-sensitive element is constituted of a heater board and a solid electrolyte pellet, and is held suspended in air in the cover by connecting a reference electrode and a detection electrode provided respectively on the heater board side and on the side opposite thereto of the solid electrolyte pellet to a pair of electrode pins using lead wires and supporting the electrode pins passing through a base.

Patent document 1: JP H3-162658A
Patent document 2: JP 2006-126160A
Patent document 3: JP 2006-47230A

### DISCLOSURE OF THE INVENTION

### PROBLEMS TO BE SOLVED BY THE INVENTION

However, in each of these conventional gas sensors, the electrode pins of the gas-sensitive element, or further electrode pins of the compensating element, pass through the mounting base and are supported therein, and their respective base end portions vertically project from the rear surface of the mounting base by a fixed length. Therefore, the electrode pins are close to each other, and their material is generally difficult to be soldered such as Hastelloy that is a kind of stainless steel, causing a problem of a poor workability of wiring between the electrode pins and the detection circuit. Further, the degree of freedom (flexibility) when the gas sensor is installed in a device is low, and an excessive space is required in some case.

The invention is made to solve the above problems and a first object thereof is to facilitate the work of wiring the gas-sensitive element to the electrode pins, or further the work of wiring the compensating element to the electrode pins, to increase the flexibility when installing in a device and to reduce the space.

Further, in the conventional gas sensor, since each electrode pin is directly or a pin stay firmly fixed to a middle portion of the electrode pin is press-fitted into a through-hole formed in the mounting base or bonded with a glass adhesive or the like to be fixed in the mounting base, the gas-sensitive element or the compensating element or the pin stay or the mounting base may be broken during the press-fitting in the former case and the heat resistance is insufficient in the latter case, along with a problem of poor assembly workability in both cases.

The invention solves such a problem and a second object thereof is to make it possible to easily perform the work of fixing the electrode pins of the gas-sensitive element and the compensating element to the mounting base without a fear of breakage and to obtain sufficient heat resistance.

### MEANS FOR SOLVING THE PROBLEMS

To achieve the first object, a gas sensor according to the invention includes: a gas-sensitive element; a plurality of electrode pins each connected to the gas-sensitive element at one end portion and supporting the gas-sensitive element; a mounting base made of an insulating material supporting the plurality of electrode pins each passing through the mounting base from one surface to another surface; and a gas-permeable cover member firmly fixed to the mounting base to cover a region on the one surface side of the mounting base including the gas-sensitive element, wherein lead-out leads extending in a direction parallel to the another surface of the mounting base are provided respectively to connect with portions of the plurality of electrode pins projecting to the another surface side of the mounting base.

It is preferable in the above-described gas sensor that a spacer through which each electrode pin passes and which supports each lead-out lead and a pressing member covering substantially the entire surface of the spacer on the side supporting each lead-out lead are provided on the side of the mounting base where each electrode pin projects, and both of the pressing member and the spacer are made of an insulating material.

The gas sensor may be a gas sensor including: a gas-sensitive element unit in which a plurality of electrode pins electrically connected to a gas-sensitive element and supporting the gas-sensitive element pass through a pin stay made of an insulating material and are held in parallel; a mounting base holding the pin stay fitting therein; a gas-permeable cover member firmly fixed to one surface side of the mounting base to cover the gas-sensitive element side of the gas-sensitive element unit; and a holder having an opening from which the cover member projects, and fixing and holding the mounting base, or may be a gas sensor further including a compensating element unit in which a pair of electrode pins electrically connected to both terminals of a compensating element for temperature compensation and supporting the compensating element pass through a second pin stay made of a heat resistant insulating material and are held in parallel.

In this case, lead-out leads extending in a direction parallel to another surface of the mounting base are provided respectively to connect with the electrode pins of the gas-sensitive element unit, or further with the electrode pins of the compensating element unit, projecting to the another surface side of the mounting base.

It is preferable that a tip end face of the electrode pin and a flattened portion of the lead-out lead are in contact with each other, and contacting portions of the electrode pin and the lead-out lead are connected by laser welding.

Alternatively, an outer peripheral surface of a portion of the electrode pin projecting from the mounting base and an outer peripheral surface of a portion of the lead-out lead in parallel to the electrode pin made by bending at one end portion may be in contact with each other along respective center axes, and contacting potions of the electrode pin and the lead-out lead may be connected by laser welding.

It is preferable that a spacer through which each electrode pin passes and which supports each lead-out lead and a pressing member covering substantially the entire surface of the spacer on the side supporting each lead-out lead are provided on the side of the holder where each electrode pin projects, and both of the pressing member and the spacer are made of an insulating material.

It is more preferable that at the holder, a plurality of spacer holding pieces holding an outer peripheral surface of the spacer and a plurality of pressing member locking pieces locking the pressing member by pressing the pressing member toward the spacer side are integrally formed of a metal plate with spring properties.

It is preferable in the gas sensor provided with the spacer and the pressing member that a groove guiding each lead-out lead is formed in a surface of the spacer on the side supporting each lead-out lead or in a surface of the pressing member on a side in contact with the spacer.

It is preferable that a plurality of the grooves are formed in each of directions perpendicular to each other, and each lead-out lead connected to each electrode pin has a bent portion at least one place along the groove.

Further, a plurality of grooves or projections and depressions (protruding portions and/or recessed portions or an uneven surface) for increasing the surface area may be formed on a surface of the pressing member on a side not in contact with the spacer.

It is more preferable that at least one of the spacer and the pressing member is made of ceramics or porous ceramics. Further, it is preferable that the cover member is also made of porous ceramics and the mounting base is made of ceramics or porous ceramics.

It is desirable that the mounting base has a fitting slot (fitting slots) for fitting the pin stay or the first pin stay and the second pin stay therein, and the fitting slot is in an opening shape equal to or slightly larger than the outer peripheral shape of the stay from the another surface side of the mounting base to a middle in the thickness direction, and the opening shape is reduced in size from the middle to the one surface side to form a stepped part, and that a cutout part (cutout parts) being an escape (escapes) for the gas-sensitive element or for the gas-sensitive element and the compensating element to pass through when the pin stay(s) is(are) fitted in the fitting slot(s) is(are) formed in the stepped part(s).

Further, it is desirable that a pressing spring pressing the pin stay or the first pin stay and the second pin stay fitted in the fitting slot(s) against the stepped part(s) by pressing the pin stay(s) from a rear surface (rear surfaces) thereof is interposed between the holder and the mounting base.
It is desirable in the gas sensor provided with the gas-sensitive element and the compensating element that a heat shielding plate for thermally shielding the gas-sensitive element and the compensating element in the cap is provided at the base. It is preferable that the heat shielding plate is made of ceramics or porous ceramics.

### EFFECT OF THE INVENTION

The gas sensor according to the invention is provided with lead-out leads extending in a direction parallel to the rear surface of the mounting base which respectively connect with at least the electrode pins of the gas-sensitive element projecting from the rear surface of the mounting base, and can therefore optimize the interval, the arrangement, the length, the material and so on of the lead-out leads in consideration of workability, thereby facilitating the work of wiring the gas-sensitive element, or further the compensating element, to the electrode pins. Further, it is possible to increase the flexibility when installing in a device and reduce the space behind the gas sensor to achieve space saving.

Further, one or a plurality of fitting slots each having a stepped part formed at a middle in the thickness direction are provided in the mounting base, and a pressing spring pressing the pin stay of the gas-sensitive element unit, or further the pin stay of the compensating element unit, fitted in the fitting slot(s) against the stepped part(s) by pressing the pin stay(s) from the rear surface(s) thereof is interposed between the holder and the mounting base, whereby the pin stay(s) can be fixed and held in the mounting base by the pressing force of the pressing spring without press-fitting or bonding the pin stay(s) in the fitting slot(s).

Further, a cutout part or cutout parts each being an escape for the gas-sensitive element, or further the compensating element, to pass when the pin stay(s) is(are) fitted in the fitting slot(s) is(are) formed at the stepped part(s), thereby eliminating the possibility of the gas-sensitive element or/and the compensating element bumping into the stepped part(s) to break when the gas-sensitive element unit or/and the compensating element unit is(are) inserted through the fitting slot(s) to be fixed and held in the mounting base.

### BRIEF DESCRIPTION OF DRAWINGS

FIG 1 is a perspective view showing the appearance of a preferred embodiment of a gas sensor according to the invention;
FIG. 2 is a perspective view showing the gas sensor upside down;
FIG. 3 is an exploded perspective view of the holder of the gas sensor and parts on the detecting part side fixed and held by the holder as seen from the same direction as in FIG 2;
FIG. 4 is an exploded perspective view of the holder, the electrode pins projecting from the rear surface of the holder, the lead-out leads, the spacer, and the pressing member;

FIG. 5 is a sectional view along the long side direction of the holder showing the attachment state of the gas sensor to shown in FIG. 1 and FIG. 2 to the device;
FIG. 6 is a sectional view along the short side direction of the same holder;
FIG 7 is a sectional view showing the same section as in FIG. 5 in the state that the cap, the heat shielding plate, and the attachment portion to the device are omitted and the pressing spring is interposed between the holder and the mounting base;
FIG 8 is a rear view of the same showing the state that the pressing member is removed;
FIG. 9 is a side view showing the attachment state of the gas sensor to the device shown in FIG 5;

FIG. 10 is an enlarged perspective view of the mounting base as seen from the rear surface side;
FIG. 11 is a plan view of the same;
FIG. 12 is a sectional view taken along the line A-A in FIG 11;
FIG. 13 is an enlarged plan view of the pressing spring;
FIG. 14 is an end view of the section taken along the line B-B in FIG. 13;
FIG. 15 is an end view of the section taken along the line C-C in FIG 13;
FIG 16(a) is an enlarged front view of a portion near the connecting end part of the lead-out lead connected to one electrode pin and (b) is a plan view of the same;
FIG. 17(a) is an enlarged front view of a portion near the connecting end part of the lead-out lead connected in common to two electrode pins and (b) is a plan view of the same;

FIG 18 is a plan view of a surface of the pressing member in a different example on the side not in contact with the spacer;
FIG. 19 is a plan view of a surface of the same pressing member on the side in contact with the spacer;
FIG. 20 is a sectional view taken along the line D-D in FIG 18;
FIG 21 is an exploded perspective view of another preferred embodiment of the gas sensor according to the invention;
FIG 22 is an enlarged perspective view of a pressing member in the embodiment;
FIG 23 is a sectional view taken along the line E-E in FIG. 22;
FIG 24 is a perspective view of the assembly complete state of the embodiment shown in FIG 21;
FIG. 25 is an enlarged rear view showing the sate that the pressing member of the same is removed;

FIG 26 is a perspective view showing essential parts of the holder and the appearance on the side of the connected portions between the electrode pins and the lead-out leads in still another referred embodiment of the gas sensor according to the invention;
FIG. 27 is a perspective view showing the state that a dual-partitioned spacer of the same is attached;
FIG 28 is a perspective view showing the state that the pressing member is further attached; and
FIG. 29 is an enlarged explanatory view showing the internal structure of porous ceramics.

### REFERENCE OF NUMRLALS

| | |
|---|---|
| 1: holder | |
| 2: mounting base | 3: cap (cover member) |
| 4: spacer | 4a: through-hole |
| 4b: groove | 4c: recessed part |
| 6: pressing member | 6a: cutout |
| 6b: elongate protrusion | 7: gas-sensitive element unit |
| 8: compensating element unit | 9: heat shielding plate |
| 10: pressing spring | 10a: pin stay pressing leaf spring |
| 10b: mounting base pressing leaf spring | |
| 10c: punched hole | 10d: cutout |
| 11: holder board | 11 a: circular opening |
| 11b: annular projecting part | 11c: attachment hole |
| 12: holder with spring properties | |
| 12a: spacer holding piece | 12b: pressing member locking piece |
| 12b₁: locking piece part | 12c: sector part |
| 12d: through-hole | |
| 16, 26: pressing member | 16a: cutout |
| 16b: elongate projection | 16c: groove for increasing surface area |
| 21: slot for fitting heat shielding plate | |
| 21a: inlet part | 22, 23: slot for fitting pin stay |
| 22a, 23a: fitting part | 22b, 23b: through-hole part |
| 22c, 23c: stepped part | 22d, 23d: cutout part |
| 26d: protruding part | 30: casing panel of device |
| 30a: fitting hole | 30b: attachment hole |
| 31: attaching screw | 41: one half part of spacer |
| 42: other half part of spacer | |
| 41 a. 42a: half part of escape hole | |
| 51-53, 52', 53': lead-out lead | |
| 51a, 51b, 52a, 53a: connecting portion | |
| 55-58: lead-out lead | |
| 55a, 56a, 57a, 58a: connecting portion | |
| 60: pressing member | 60b: groove |
| 70: gas-sensitive element | 71, 72: electrode pin |
| 73: first pin stay | 74, 75: pin base |
| 80: compensating element | 81, 82: electrode pin |
| 83: second pin stay | 84, 85: pin base |

### BEST MODE FOR CARRYING OUT THE INVENTION

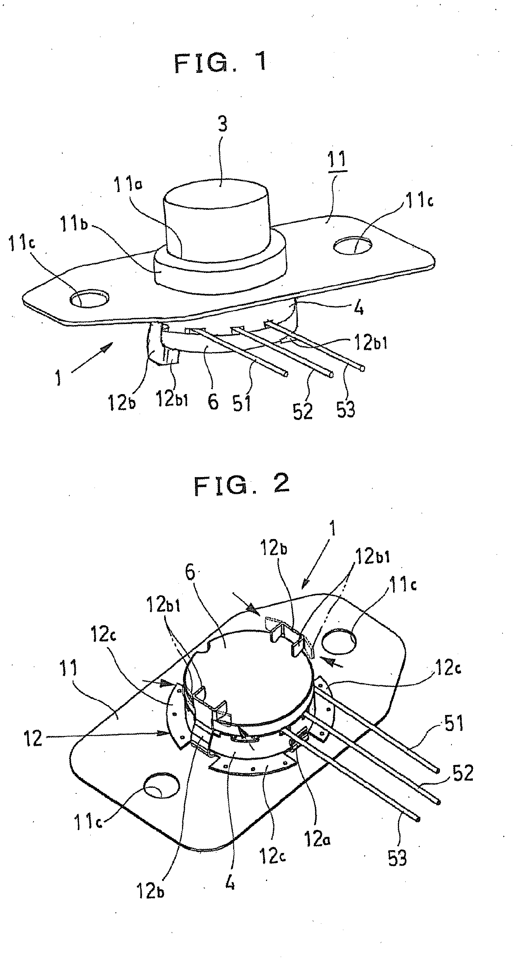

Hereinafter, the best mode for carrying out the invention will be concretely described based on the drawings.
First, the appearance of a preferred embodiment of a gas sensor according to the invention will be described using FIG. 1 and FIG. 2. This is an example of a catalytic combustion type gas sensor, and FIG. 1 is a perspective view with a gas detecting part side of the gas sensor facing upward and FIG 2 is a perspective view showing the gas sensor upside down.

In this gas sensor, it's all members are held by a holder 1 composed of a holder board 11 that is an attachment plate and a holder with spring properties 12 integrally firmly fixed on the rear surface side of the holder board 11.
The holder board 11 has an annular projecting part 11b having a circular opening 11a formed at the central portion and attachment holes 11c provided on both sides in the longitudinal direction thereof.

In the annular projecting part 11b of the holder board 11, a later-described mounting base in a circular plate shape is fitted in to be fixed and held, and a cap 3 firmly fixed to one surface of the mounting base projects from the circular opening 11a as shown in FIG 1. The cap 3 is a gas-permeable cover member and made of porous ceramics in a dome shape in this example.

In this cap 3, a gas-sensitive element being a detecting element and a compensating element for temperature compensation, which will be described later in detail, are each supported by a pair of electrode pins passing through a pin stay with both terminals of the gas-sensitive element or the compensating element connected to the pair of electrode pins, and are fixed and held on the mounting base via the respective electrode pins and pin stays and arranged to face each other.

Meanwhile, the electrode pins (four pins) project on the rear surface side of the holder 1, and are inserted through the spacer 4 and connected to lead-out leads 51 to 53 made of stainless steel extending in a direction parallel to the rear surface of the holder 1 on the upper surface side in FIG 2 (also parallel to the other surface of the mounting base). In this example, two electrode pins each connected to one terminal of each of the gas-sensitive element and the compensating element, of the four electrode pins, are connected in common to one lead-out lead 51, and the other two electrode pins are individually connected to the two lead-out leads 52, 53.

On the rear surface side of the holder with spring properties 12 which is provided on the side of projecting the electrode pins of the holder 1, a spacer 4 in a circular plate shape through which the electrode pins pass and which guides and supports the lead-out leads 51 to 53 and a pressing member 6 in a circular plate shape which covers substantially the entire surface of the spacer 4 on the side supporting the lead-out leads are provided and positioned and held by the holder 12 with spring properties.

Therefore, on the holder with spring properties 12, a pair of spacer holding pieces (only one piece is shown in FIG. 2) 12a holding the outer peripheral surface of the spacer 4 at opposed positions and a pair of pressing member locking pieces 12b locking the pressing member 6 by pressing the pressing member 6 toward the spacer side at positions opposed to each other are integrally formed of a metal plate with spring properties. The holder with spring properties 12 has sector parts 12c at periphery thereof integrally welded to the holder board 11.

When the pressing member 6 is overlaid on the spacer 4, and locking piece parts 12b₁ formed on both sides of a tip portion of each of the pressing member locking pieces 12b of the holder with spring properties 12 are then bent at substantially the right angle in directions shown by arrows in FIG. 2, the locking piece parts 12b₁ come into contact with the rear surface of the pressing member 6 and hold the pressing member 6 by pressing the pressing member 6 to the spacer 4 using the spring force enhanced by the curvature of a rising portion of each of the pressing member locking pieces 12b. The spacer holding piece 12a and the pressing member locking piece 12b of the holder with spring properties 12, not limited in number to one pair, only need to be provided at a plurality of places and may be provided at three places or more at regular intervals. Both of the spacer 4 and the pressing member 6 are preferably formed of ceramics.

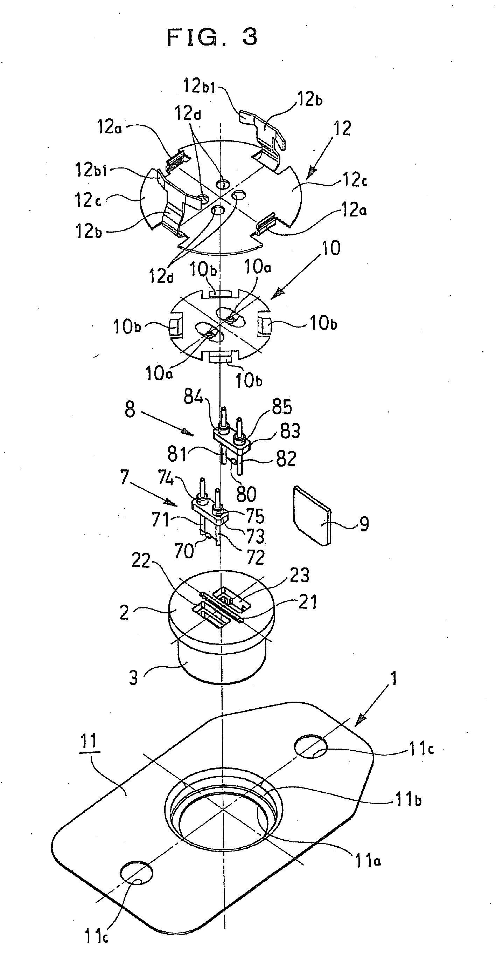

The details of the embodiment of this gas sensor will be described with reference to FIG. 3 to FIG. 17.
FIG 3 is an exploded perspective view of the holder of the gas sensor and it's parts on the detecting part side to be fixed and held on the holder as seen from the same direction as in FIG. 2, showing the holder board 11 and the holder with spring properties 12 constituting the holder 1, the mounting base 2 and the cap 3, the gas-sensitive element unit 7 and the compensating element unit 8, the heat shielding plate 9, and the pressing spring 10.

The holder board 11 is a member in the shape of a slightly elongated base of baseball formed by pressing a stainless steel plate, in which the annular projecting part 11b with the circular opening 11a at the central portion as described above is formed by drawing and the attachment holes 11c are opened on both sides in the longitudinal direction.

The mounting base 2 is in a circular plate shape, has a thin slot 21 for fitting the heat shielding plate therein formed along the diameter line of the mounting base 2, and has a pair of slots 22, 23 for fitting a pair of pin stays therein respectively formed on both sides across the slot 21. The details of the shapes will be described later.
On the lower surface side of the mounting base 2 in FIG. 3, the cap 3 that is a gas-permeable cover member in a dome shape is firmly fixed by bonding with a glass adhesive or the like.

The gas-sensitive element unit 7 includes a gas-sensitive element 70 being the detecting element, and a pair of electrode pins 71, 72 electrically connected to both terminals of the gas-sensitive element 70 and supporting the gas-sensitive element 70 which are inserted through a first pin stay 73, and annular pin bases 74, 75 provided on the upper side in FIG. 3 of the first pin stay 73 to fit on the respective electrode pins 71, 72 in a manner that a glass adhesive or the like is used to bond and fix the first pin stay 73, the electrode pins 71, 72, and the pin bases 74, 75 such as to hold the electrode pins 71, 72 in parallel. Here, the surfaces to be bonded of the electrode pins 71, 72 are partially provided with projections and depressions (by crisscross knurling in this example) to easily obtain adhesive strength.

The compensating element unit 8 includes a compensating element 80, and a pair of electrode pins 81, 82 electrically connected to both terminals of the compensating element 80 and supporting the compensating element 80 which are inserted through a second pin stay 83, and annular pin bases 84, 85 provided on the upper side in FIG. 3 of the second pin stay 83 to fit on the respective electrode pins 81, 82.

A glass adhesive or the like is used to bond and fix the second pin stay 83, the electrode pins 81, 82, and the pin bases 84, 85 such as to hold the electrode pins 81, 82 in parallel. Here, the surfaces to be bonded of the electrode pins 81, 82 are partially provided with projections and depressions (by crisscross knurling in this example) to easily obtain adhesive strength.

All of the mounting base 2, the first pin stay 73 and the pin bases 74, 75 of the gas-sensitive element unit 7, the second pin stay 83 and the pin bases 84, 85 of the compensating element unit 8, and the heat shielding plate 9 are made of a heat resistant insulating material, preferably ceramics. Depending on usage, the mounting base 2 and the heat shielding plate 9 may be formed of porous ceramics. The electrode pins 71, 72, 81, 82 are made of a conductive material with high heat resistance and high corrosion resistance, for example, Hastelloy that is a kind of stainless steel.

In the gas-sensitive element 70 being the detecting element, a heater coil made of a platinum-based alloy wire is buried in a heat conducting layer whose surface is coated with or carries a oxidation catalyst causing combustion of a detection target gas brought into contact therewith, and both ends of the heater coil are connected to the electrode pins 71, 72. The compensating element 80 is an element provided to compensate the influence by a change in ambient temperature and has a heater coil having the same electric characteristics as those of the heater coil of the gas-sensitive element 70 buried in a heat conducting layer having no oxidation catalyst, and both ends of the heater coil are connected to the electrode pins 81, 82.

As described above, the holder with spring properties 12 the annular projecting part 11b respectively is formed such that a pair of spacer holding pieces 12a and a pair of pressing member locking pieces 12b each having curvature halfway of a rising portion are integrally formed of a metal plate (a stainless steel plate in this example) in a circular plate shape with spring properties at intervals of 90° in the circumferential direction.

Further, four through-holes 12d are formed at the central portion of the circular plate shape, which portions of the electrode pins 71, 72, 81, 82 of the gas-sensitive element unit 7 and the compensating element unit 8 projecting from the rear surface of the mounting base 2 can be inserted through and the pin bases 74, 75, 84, 85 can be fitted in. At peripheral portions between the spacer holding pieces 12a and the pressing member locking pieces 12b, four sector parts 12c are formed.

The pressing spring 10 is a member interposed between the mounting base 2 and the holder with spring properties 12, in which four pieces of leaf springs 10a in pairs pressing the first pin stay 73 and the second pin stay 83 respectively from the rear surfaces thereof against later-described stepped parts are formed, together with a plurality of (four in this example) leaf springs 10b pressing at a plurality of places (four places in this example) symmetrical about a center near the outer periphery of the rear surface of the mounting base 2, by cutting and raising a sheet of leaf spring material (a stainless steel plate in this example).

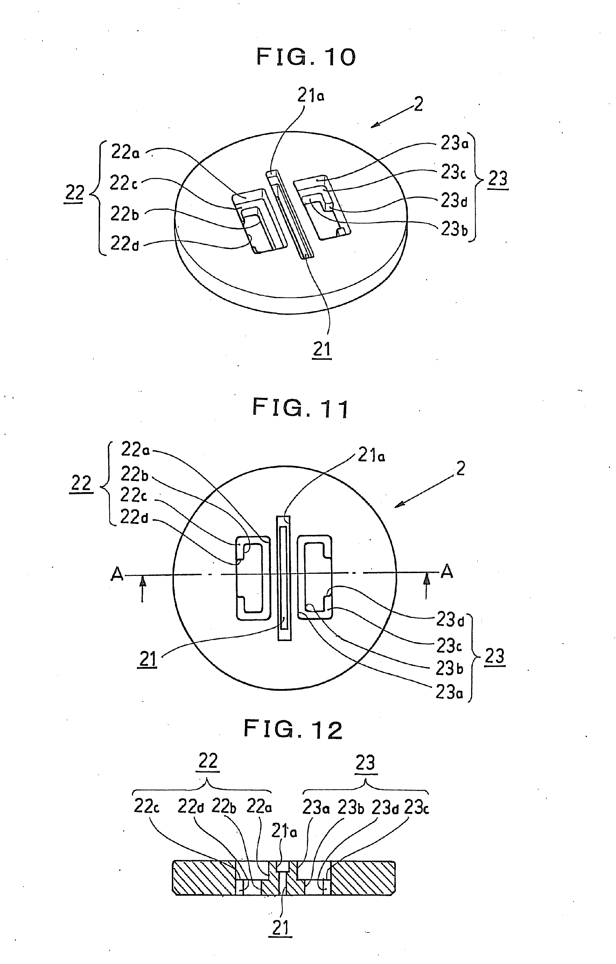

Here, the enlarged details of the mounting base 2 are shown in FIG 10 to FIG. 12. FIG. 10 is a perspective view of the mounting base as seen from the rear surface side. FIG. 11 is a plan view of the same, and FIG 12 is a sectional view taken along the line A-A in FIG. 11.

In the slot 21 for fitting the heat shielding plate therein that is formed to be elongated along the diameter of the mounting base 2, an inlet part 21 a on the rear surface side of the mounting base 2 has a thickness and a length larger than those of the heat shielding plate 9 shown in FIG. 3 and has a shape of the back into which the heat shielding plate 9 can be inserted. An adhesive may be filled into the inlet part 21a after the heat shielding plate 9 is inserted into the slot 21, or the base end portion of the heat shielding plate 9 may be shaped to fit in the inlet part.

Further, the slots 22, 23 for fitting a pair of pin stays formed in parallel on both sides across the slot 21 for fitting the heat shielding plate therein are formed such that fitting parts 22a, 23 a from the rear surface side of the mounting base 2 to almost the middle portion halfway in the thickness direction are in opening shapes equal to or slightly larger than the outer peripheral shapes of the first and second stays 73, 83, the opening shapes are reduced in size from the middle to the through-hole parts 22b, 23b on the front surface side (the lower surface in these drawings), and stepped parts 22c, 23c are formed between the fitting parts 22a, 23a and the through-hole parts 22b, 23b.

In the stepped parts 22c, 23c, cutout parts 22d, 23d being escapes for the gas-sensitive element 70 and the compensating element 80 to be able to easily pass through when the gas-sensitive element unit 7 and the compensating element unit 8 are inserted through the slots 22, 23 respectively and the first pin stay 73 and the second pin stay 83 are fitted therein, are further formed along long sides thereof on the outer sides distantly from each other.

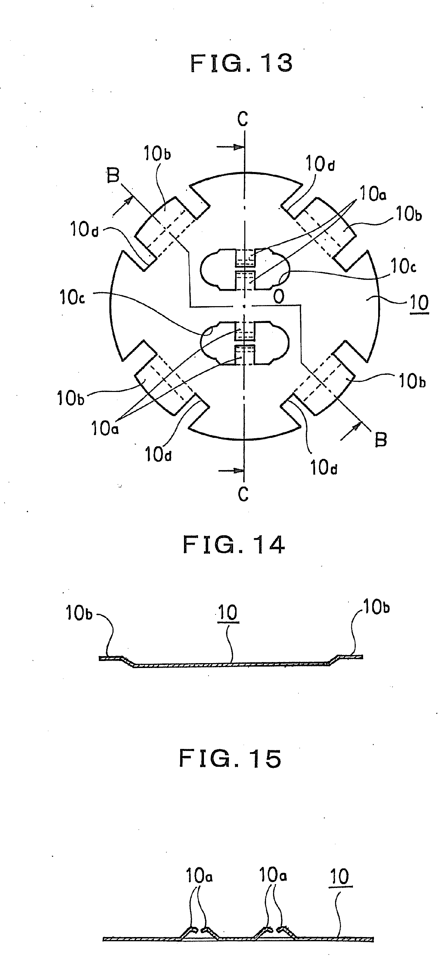

Next, the enlarged details of the pressing spring 10 are shown in FIG. 13 to FIG. 15. FIG 13 is a plan view of the pressing spring, FIG 14 is a sectional view taken along the line B-B in FIG 13, and FIG. 15 is an end view of the section taken along the line C-C in FIG. 13.

The pressing spring 10 is formed by stamping out a planar shape to obtain the shape shown in FIG 13 from a leaf spring material in a circular plate shape and forming four pieces of leaf springs 10a in pairs by punched holes 10c at symmetrical positions near the center O along the diameter line (the C-C line) and forming four pieces of leaf springs 10b at the outer peripheral portion at point symmetrical positions about the center O along diameter lines shifted by ±45° with respect to the diameter line. Each of the leaf springs 10b is formed by punching cutouts 10d in parallel from the outer periphery on both sides in the circumferential direction thereof.

The leaf springs 10a for pressing the pin stay are cut and raised along the broken lines shown in FIG 13 to be bent as shown in FIG. 15 and thereby imparted spring properties. The leaf springs 10b for pressing the mounting base are also cut and raised along the broken lines shown in FIG. 13 to be bent as shown in FIG. 14 and thereby imparted spring properties.

When the all parts shown in FIG. 3 are assembled, the gas-sensitive element 70 side of the gas-sensitive element unit 7 and the compensating element 80 side of the compensating element unit 8 are inserted through the slots 22, 23 of the mounting base 2 respectively, and the first pin stay 73 and the second pin stay 83 are fitted into the fitting parts 22a, 23a of the slots 22, 23 show in FIG. 10 to FIG. 12 and locked on the stepped parts 22c, 23c. Thus, the gas-sensitive element unit 7 and the compensating element unit 8 can be held on the mounting base 2 facing each other.

Then, the heat shielding plate 9 is inserted into the slot 21 of the mounting base 2 in a manner to project and interpose in the cap 3 to divide between the gas-sensitive element unit 7 and the compensating element unit 8, thereby thermally shielding them. It is preferable to fill an adhesive into the inlet part 21 a of the slot 21 after the heat shielding plate 9 is inserted into the slot 21 to fix the heat shielding plate 9.

The cap 3 of the sensor part constituted by assembling the parts as described above is inserted through the circular opening 11 a of the holder board 11, the mounting base 2 is fitted into the annular projecting part 11b, the pressing spring 10 is mounted on the mounting base 2, and the holder with spring properties 12 is further mounted. These all parts are assembled with the center axes shown by a one-dotted chain line in FIG. 3 and radial directions perpendicular thereto aligned with one another.

In this state, the rear end portions of the electrode pins 71, 72, 81, 82 of the gas-sensitive element unit 7 and the compensating element unit 8 where the pin bases 74, 75, 84, 85 are fitted project from the rear surface of the mounting base 2, and project from the four through-holes 12d of the holder with spring properties 12 via the punched holes 10c shown in FIG. 13 of the pressing spring 10 respectively. Then, the sector parts 12c of the holder with spring properties 12 are firmly fixed by spot welding or laser-welding to the holder board 11.

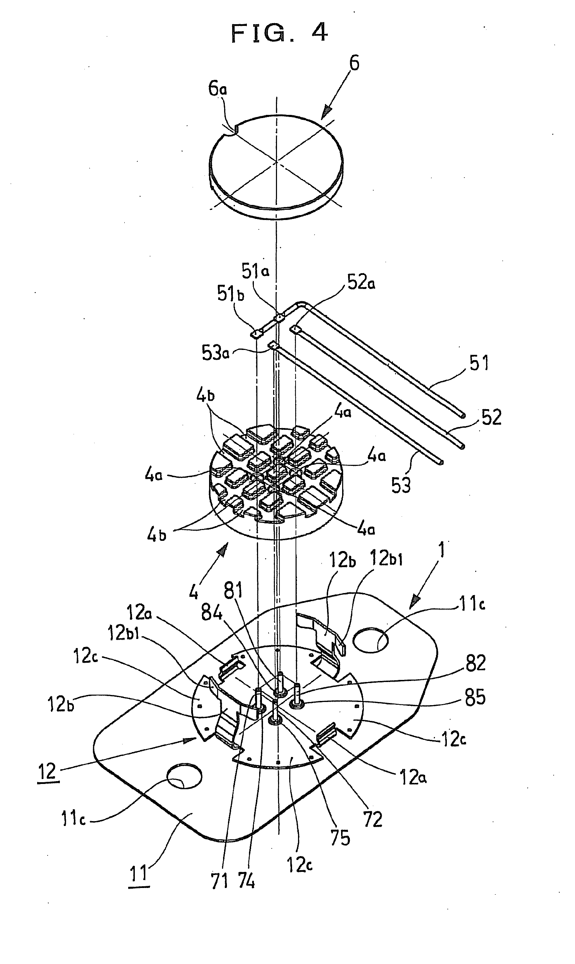

Next, members provided on the rear surface side of the holder will be described using FIG. 4. FIG. 4 is an exploded perspective view showing the state in which the parts are assembled on the holder 1 composed of the holder board 11 and the holder with spring properties 12 shown in FIG 3 and the sector parts 12c of the holder with spring properties 12 are firmly fixed by welding to the holder board 11, together with the spacer 4, the lead-out leads 51 to 53 and the pressing member 6 shown in FIG. 2.

The electrode pins 71, 72, 81, 82 of the gas-sensitive element unit 7 and the compensating element unit 8 are insulated by the pin bases 74, 75, 84, 85 and project from the through-holes 12d of the holder with spring properties 12 respectively.

In the spacer 4, four through-holes 4a which the electrode pins 71, 72, 81, 82 pass through are provided, and a plurality of grooves 4b perpendicular to each other are formed in the surface on the upper side in FIG. 4. Therefore, the spacer 4 supports the lead-out leads 51 to 53 and also functions as guide members guiding the lead-out leads 51 to 53 using the plurality of grooves 4b.

In the pressing member 6 covering the spacer 4, small semicircular cutouts 6a are formed at predetermined positions on the outer peripheral surface and are used as positioning indications with respect to the spacer 4. Both of the spacer 4 and the pressing member 6 are made of a heat resistant insulating material, preferably, ceramics or porous ceramics.

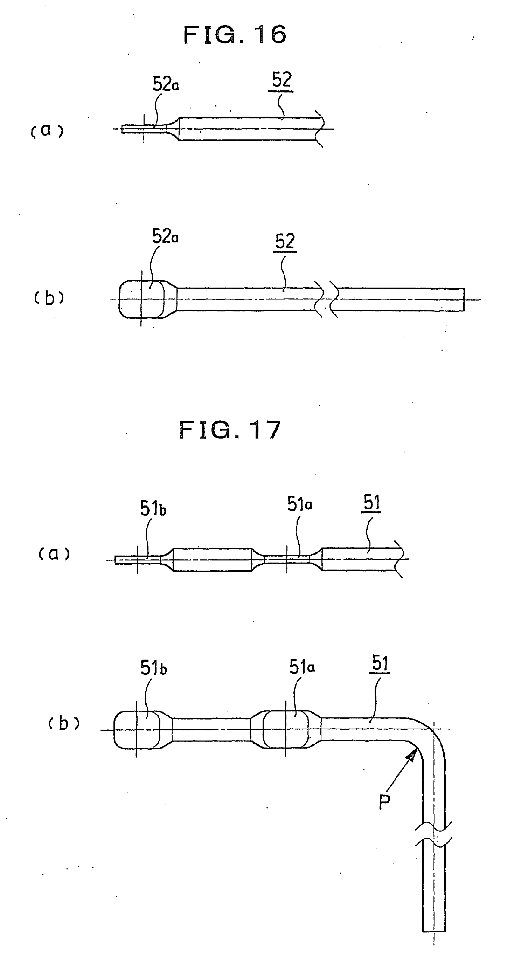

The lead-out leads 51 to 53 will be described here with reference also to FIG 16 and FIG. 17. FIG 16 is an enlarged view of a portion near the connecting end part of the lead-out lead 52 connected to one electrode pin, and FIG. 17 is an enlarged view of a portion near the connecting end part of the lead-out lead 51 connected in common to two electrode pins, in each of which (a) is a front view and (b) is a plan view.

The lead-out leads 51 to 53 are produced by cutting, for example, a wire made of stainless steel, and the lead-out lead 52 forms a straight line shape as shown in FIG. 16 in which a flat connecting portion 52a is formed at one end portion. Also in the lead-out lead 53, a flat connecting portion 53a shown in FIG. 4 is formed, as in the lead-out lead 52. Further, as the material of the lead-out leads 51 to 53, Monel alloy (for example, containing about 30 % of copper in nickel as a main component) may be employed, in which case general soldering work becomes possible so that the lead-out leads 51 to 53 can be directly soldered to the external control circuit or the like.

In the lead-out lead 51, as shown in (b) of FIG 17, one end side from a portion shown by an arrow P is bent at almost right angle, and flat connecting portions 51a and 51 b as shown in (a) are formed at a middle portion and at an end portion of the bent portion. These flat connecting portions 51a, 51b, 52a, 53a can be easily formed by pressing work to the portions in the radial direction or cutting work to the portions.

When the all parts shown in FIG. 4 are assembled, the spacer 4 is mounted on the holder with spring properties 12 while the electrode pins 71, 72, 81, 82 of the gas-sensitive element unit 7 and the compensating element unit 8 are inserted through the through-holes 4a as shown by the one-dotted chain lines, and the outer peripheral surface thereof is held by the pair of spacer holding pieces 12a, 12a. Thus, the end portions of the electrode pins 71, 72, 81, 82 slightly project into the grooves 4b of the spacer 4.

Then, the lead-out leads 51 to 53 are introduced into the grooves 4b of the spacer 4, the flat connecting portions 51 a, 51b of the lead-out lead 51 are brought into contact with the end faces of the electrode pins 71, 81, and the contact portions are connected by the laser welding. Further, the connecting portions 52a, 53a of the lead-out leads 52, 53 are brought into contact with the end faces of the electrode pins 82, 72 respectively, and the contacted portions are connected by the laser welding.

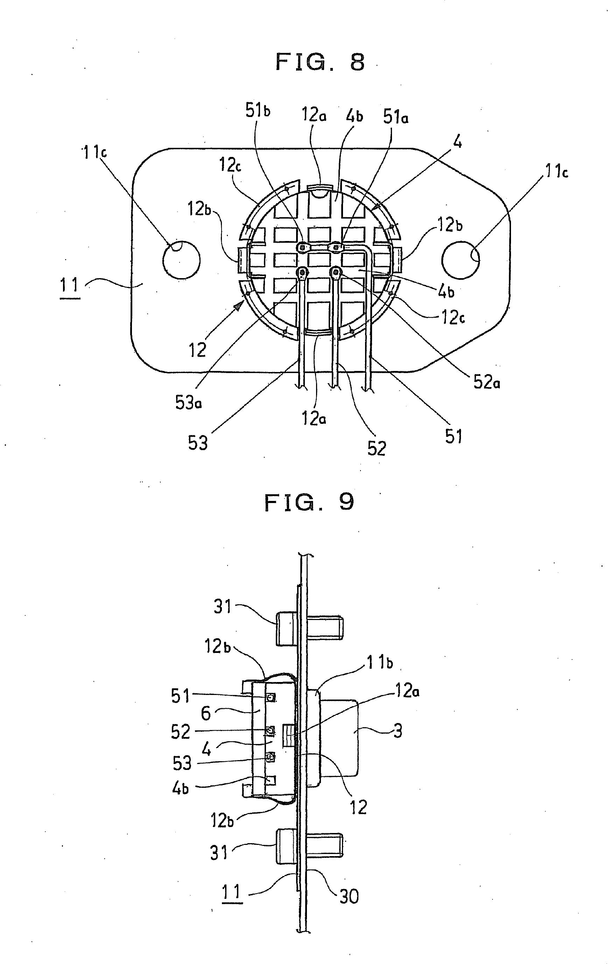

The connection complete state between the electrode pins and the lead-out leads is shown in FIG. 8. Since the electrode pins 71, 72, 81, 82 are inserted through and fixed in the through-holes 4a of the spacer 4 and the lead-out leads 51 to 53 are positioned in the grooves 4b of the spacer 4 in this way, the laser welding at the connecting portions 51a, 51b, 52a, 53a can be easily performed.

Since the connecting portions of each lead-out lead with the electrode pin is processed to be flat in this embodiment and is thus increased in contact area with the end face of the electrode pin and becomes stable, the connection by the welding can be surely performed. However, this is not essential, but the connecting portions of each of the lead-out leads may be not processed but kept in the cylindrical shape. The sectional shape of the wire itself of the lead-out lead material may be in a square shape or a flat shape other than the circular shape. Alternatively, each electrode pin end face may be formed into a recessed cylindrical surface.
Further, the connecting method is not limited to the laser welding, but measures of electric resistance welding, bonding using a conductive adhesive, swaging or the like may be employed.

By electrically and mechanically connecting the electrode pins 71, 72, 81, 82 to the lead-out leads 51 to 53 on the spacer 4 and then mounting the circular pressing member 6 to cover substantially the entire surface of the spacer 4 on the side supporting the lead-out leads 51 to 53 and bending the locking piece parts 12b₁ of the pair of pressing member locking pieces 12b, 12b of the holder with spring properties 12 inward by about 90° , the pressing member 6 is positioned in the radial direction and fixed pressed against the spacer 4, resulting in the assembly complete state shown in FIG. 1 and FIG. 2.

A ceramic adhesive, a glass adhesive or the like is filled into the gap between the grooves 4b of the spacer 4 and the pressing member 6 to firmly fix the spacer 4 and the pressing member 6 in which the electrode pins and the connecting portions of the lead-out leads are fixed.

In this embodiment, portions near the connected portions between the electrode pins 71, 72, 81, 82 and the lead-out leads 51 to 53 can be held and fixed on the mounting base and the holder board 11 by the holder with spring properties 12, the spacer 4, and the pressing member 6.

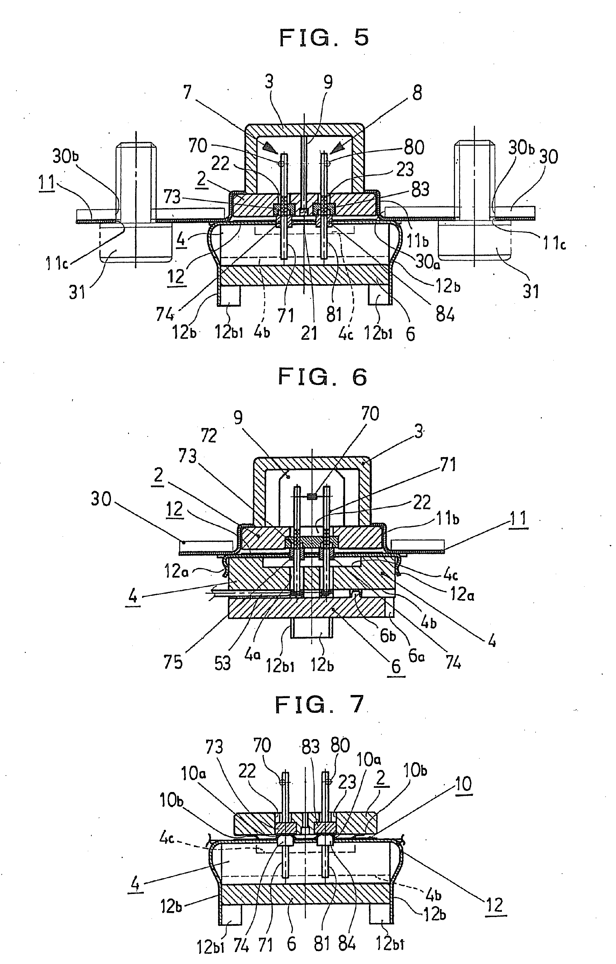

FIG. 5 to FIG. 7 are sectional views showing the assembly complete state of the gas sensor, FIG 5 is a sectional view along the long side direction of the holder showing the attachment state of the gas sensor to a device, FIG. 6 is a sectional view along the short side of the holder, and FIG. 7 is a sectional view showing the same section as in FIG. 5 in the state that the cap, the heat shielding plate, and the attachment portion to the device are omitted and the pressing spring is interposed between the holder and the mounting base which are omitted in FIG 5 and FIG. 6. FIG. 9 is a side view showing the attachment state of the gas sensor to the device shown in FIG. 5.

Though almost all of the members shown in the drawings have been already described, a newly shown part is 30 in FIG. 5 that is a casing panel of a detection target device, and the annular projecting part 11b of the holder board 11 is inserted into a fitting hole 30a to project the cap 3 therein, and attaching screws 31 are inserted through attachment holes 11c of the holder board 11 and screwed into attachment holes 30b formed with female screws of the casing panel 30 and fastened and fixed to thereby fix the gas sensor to the device. The attachment holes 30b are bored and simultaneously formed with female screws by burring work of the casing panel 30.

Further, the spacer 4 has a circular recessed part 4c formed in the surface in contact with the flat surface portion of the holder with spring properties 12 and is brought into contact with the flat surface of the holder 12 with spring properties only near the outer peripheral portion to reduce the heat conduction. Depending on the presence or the size of the recessed parts 4c, the heat conductivity from the holder with spring properties 12 to the spacer 4 can be adjusted.

Further, one elongate protrusion 6b is formed on the surface of the pressing member 6 on the side in contact with the spacer 4 as shown in FIG. 6, and fits in one of the grooves 4b of the spacer 4 to facilitate relative positioning between the spacer 4 and the pressing member 6.

Though illustration is omitted in FIG. 5 and FIG. 6, the pressing spring 10 shown in FIG. 13 to FIG. 15 is actually interposed between the flat surface portion of the holder with spring properties 12 and the mounting base 2 as shown in FIG. 7. Therefore, the elasticity of the leaf springs 10a in pairs for pressing the pin stay formed on the pressing spring 10 is used to press the portions between the electrode pins of the pin stays 73, 83 of the gas-sensitive element unit 7 and the compensating element unit 8 against the stepped parts 22c, 23 c of the slots 22, 23 shown in FIG. 10 to FIG. 12.

Therefore, the pin stays 73, 83 can be surely fixed even if they are not press-fitted into the slots 22, 23. Further, the four leaf springs 10b formed at the outer peripheral portion of the pressing spring 10 are in contact with the rear surface of the mounting base 2 and slightly pressed by the flat surface portion of the holder with spring properties 12, so that their elasticity can press the mounting base 2 against the annular projecting part 11b of the holder board 11 to surely hold the mounting base 2. In this event, a slight gap is created between the rear surface of the mounting base 2 and the flat surface portion of the holder with spring properties 12 and can greatly reduce the heat conduction.

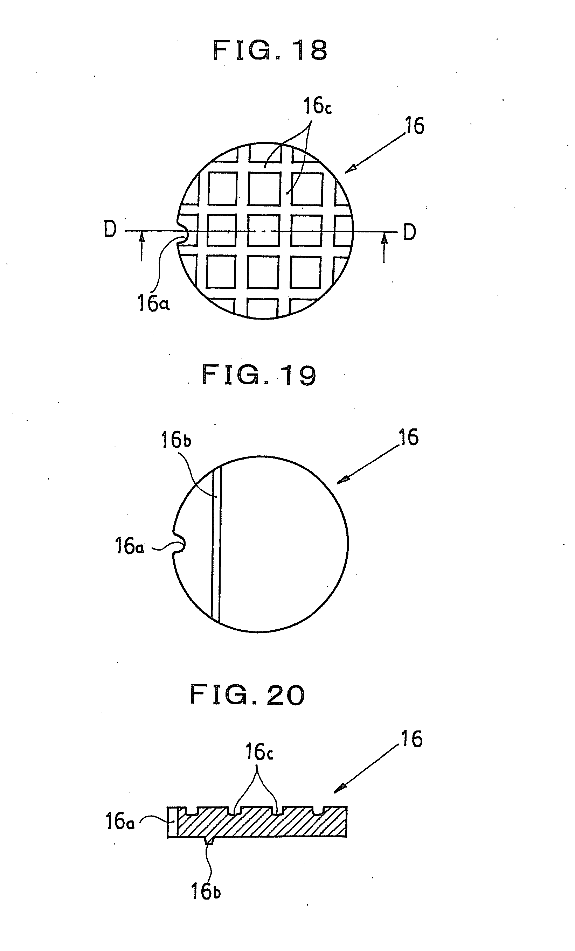

Different Example of Pressing Member
A different example of the pressing member will be described using FIG 18 to FIG 20. FIG 18 is a plan view of a surface of the pressing member on the side not in contact with the spacer, FIG 19 is a plan view of a surface on the side in contact with the spacer, and FIG 20 is a sectional view taken along the line D-D in FIG 18.

The pressing member 16 is also made of ceramics or porous ceramics similarly to the above-described pressing member 6, in which a cutout 16a for positioning is formed at a predetermined position on the outer peripheral surface and one elongate projection 16b is formed on a surface thereof on the side in contact with the spacer 4. Further, on the surface of the pressing member 16 on the side not in contact with the spacer 4, a plurality of grooves 16c for increasing the surface area are formed at regular intervals perpendicular to one another as shown in FIG 18. By changing the density and the depth of the grooves 16c, the surface area in contact with ambient air can be increased or decreased to adjust the heat release performance of the pressing member 16. In place of the grooves, many protrusions and depressions may be provided as in a later-described example.

Since this gas sensor needs to satisfy converse requirements that the gas sensor has high heat release performance (cooling performance) and good response and that the gas sensor is immune to wind and has good stability, various materials and shapes of the spacer and the pressing member were also compared and evaluated.

For example, spacers and pressing members were made of ceramics and porous ceramics containing 96 % of alumina, and the pressing member having a flat rear surface was combined with grooves formed different in density and depth, and evaluated. As a result, in the case where both of the pressing member and the spacer were made of ceramics, the pressing member having denser and deeper grooves was apt to be better in response but worse in stability, whereas in the case where both of the pressing member and the spacer were made of porous ceramics, even if the pressing member having less denser and shallower grooves was apt to be good in response and good also in stability.

### Another Embodiment of Gas Sensor

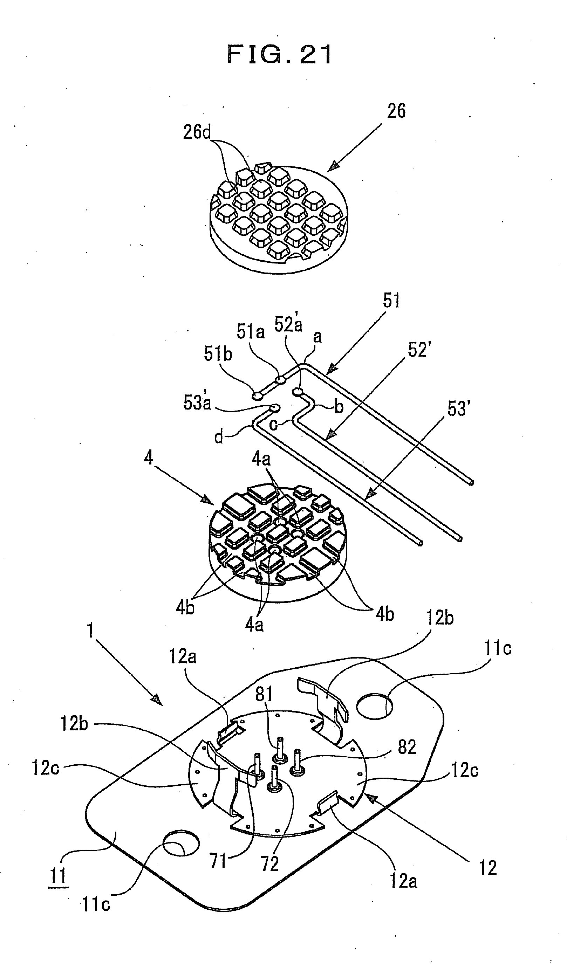

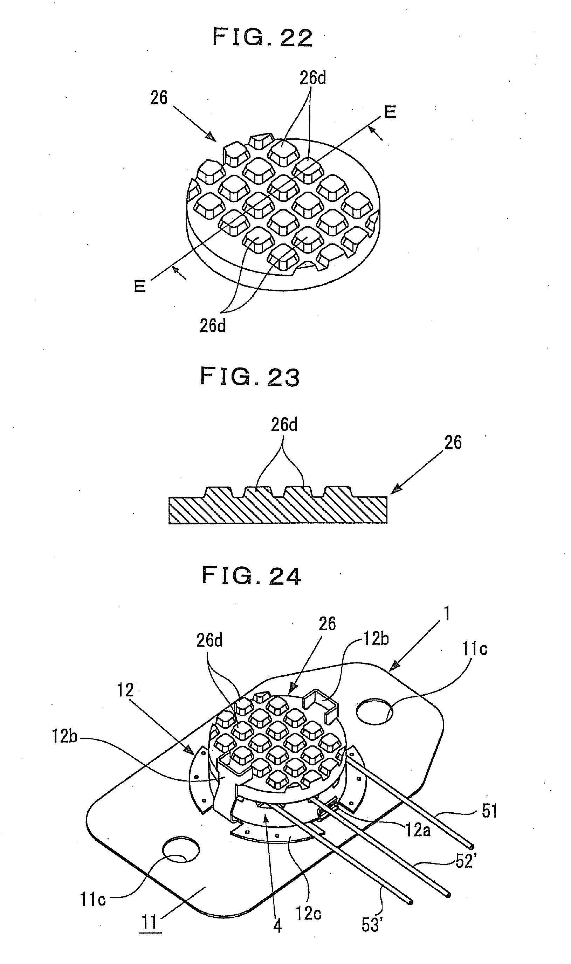

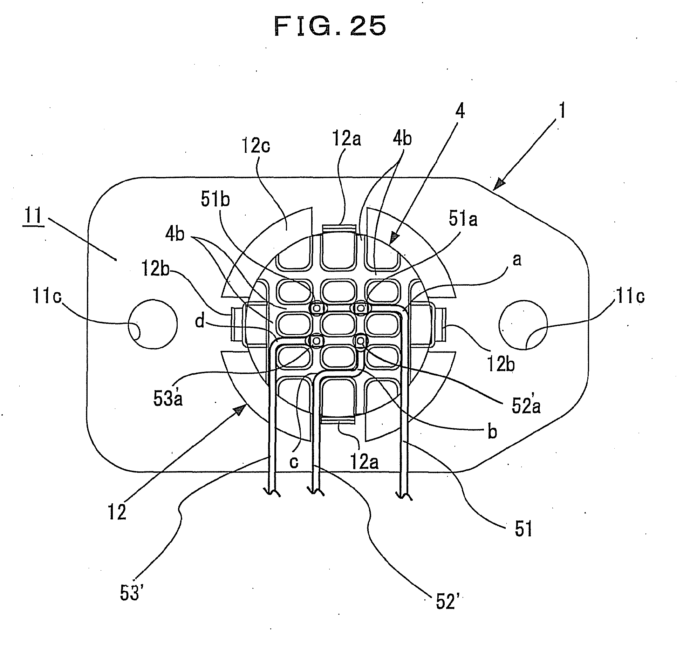

Another preferable embodiment of the gas sensor according to the invention will be described using FIG 21 to FIG 25. FIG. 21 is an exploded perspective view of the gas sensor as seen from the rear surface side. FIG. 22 is an enlarged perspective view of a pressing member used in the gas sensor, and FIG. 23 is a sectional view taken along the line E-E in FIG. 22. FIG. 24 is a perspective view of the assembly complete state of the gas sensor as seen from the rear surface side, and FIG. 25 is an enlarged rear view showing the sate that the pressing member is removed.

What are different in this embodiment from the embodiment described using FIG. 1 to FIG 15 are only the shapes of the lead-out leads and the pressing member, and the other parts in FIG. 21 to FIG. 25 are given the same reference of numerals used in FIG. 1 to FIG 15, and explanation thereof will be omitted.

The spacer 4 used in the gas sensor is the same as that used in the above-described embodiment but will be described again because the spacer 4 relates to the shapes of the lead-out leads. In the spacer 4, four through-holes 4a which the electrode pins 71, 72, 81, 82 pass through are provided, and a plurality of grooves 4b in each of directions perpendicular to one another are formed in the surface on the upper side in FIG. 21.

On the other hand, lead-out leads 51, 52', 53' are formed of stainless steel or Monel alloy as in the above-described example, and have respective bent portions a, b, c, d bent at substantially right angle at least one place along the respective grooves 4b of the spacer 4. Note that the lead-out lead 51 connected in common with the electrode pin 71 of the gas-sensitive element unit and the electrode pin 81 of the compensating element unit has the same shape as that in the above-described embodiment. Numerals 51a, 51b, 52a', 53a' denote flat connecting portions.

The lead-out leads 51, 52', 53' are fitted in the grooves 4b of the spacer 4 respectively to be supported and guided as shown in FIG. 25, and the connecting portions 51a, 51b, 52a', 53a' are connected to the electrode pins 71, 72, 81, 82 and extended to the outside in parallel to one another.

Since the bent portions a, b, c, d of the lead-out leads 51, 52', 53' are arranged along the grooves 4b of the spacer 4 as described above, side wall surfaces of the grooves 4b in which the bent portions are arranged function stoppers against the external stress applied on the lead-out leads 51, 52', 53', especially the tensile force in the same direction as the extended direction to the outside. Therefore, the stress applied on the connecting portions (welding points) with the electrode pins can be reduced, eliminating the concern about breakage.

A pressing member 26 is formed of ceramics or porous ceramics in a circular plate shape and formed with many protruding portions 26d to increase the surface area of the surface (the upper surface in FIG. 21 to FIG. 23) on the side not in contact with the spacer 4. In this embodiment, many protruding portions 26d in the same shape are formed arranged at regular intervals in a matrix as clearly shown in FIG. 21 to FIG. 23. However, it is not always necessary to form the protruding portions 26d in this manner, but protruding portions different in size and shape may be formed at random positions.

The assembly complete state of the gas sensor in this embodiment when seen from the rear surface side is as shown in FIG. 24. Since many protruding portions 26d are formed on the rear surface of the pressing member 26, the surface area is increased to improve the heat release performance. By changing the density and the height of the protruding portions 26d, the surface area in contact with ambient air can be increased or decreased to adjust the heat release performance of the pressing member 26.

Note that it is preferable to provide, also in the pressing member 26, the cutout for positioning on the outer peripheral surface and to form one elongate projection 6b on the surface thereof on the side in contact with the spacer 4 as in the pressing member 16 described using FIG 18 to FIG. 20 to facilitate relative positioning between the spacer 4 and the pressing member 26.

Further, on the rear surface of the pressing member, recessed parts may be formed in place of the protruding parts, or both of the protruding parts and the recessed parts may be formed, or the rear surface of the pressing member may be formed into an uneven surface. Further, though the plurality of grooves 4b perpendicular to one another for arranging and guiding the lead-out leads 51, 52', 53' are formed in the rear surface of the spacer 4 in this embodiment, similar grooves for arranging and guiding the lead-out leads may be formed in the surface of the pressing member 26 on the side in contact with the spacer 4.

### Still Another Embodiment of Gas Sensor

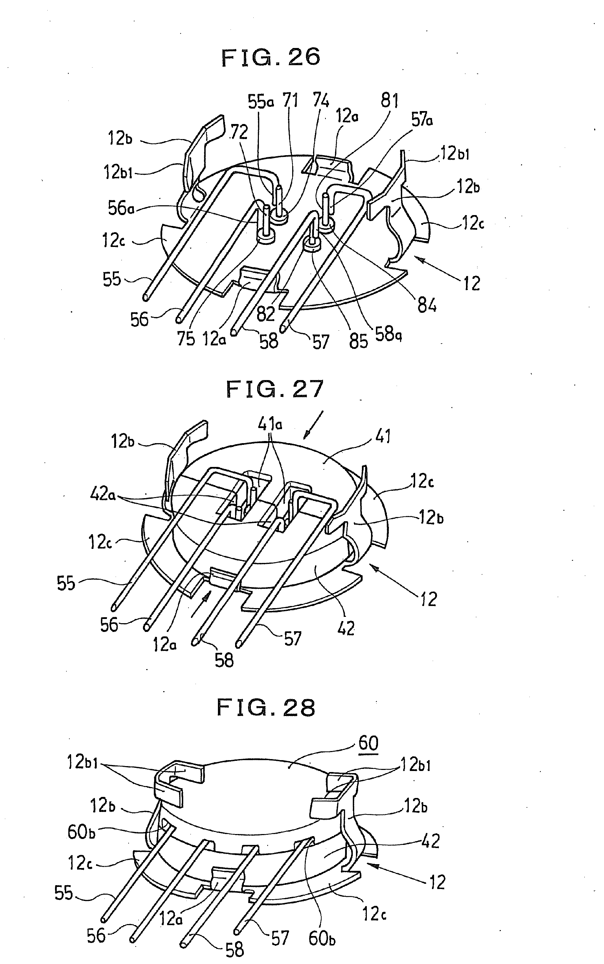

The main parts in still another preferred embodiment of the gas sensor according to the invention will be described using FIG 26 to FIG. 28. FIG. 26 is a perspective view showing the holder with spring properties of the gas sensor and the appearance on the side of the connected portions between the electrode pins and the lead-out leads, FIG. 27 is a perspective view showing the state that a dual-partitioned spacer is attached to the same, and FIG. 28 is a perspective view showing the state that the pressing member is further attached.

The holder 12 with spring properties of the gas sensor is the same as that in the above-described embodiments. What are different in this embodiment from the above-described embodiments are only the connected portions between the electrode pins and the lead-out leads and the spacer and the pressing member. In this embodiment, lead-out leads 55 to 58 are individually connected to the four electrode pins 71, 72, 81, 82 respectively.

The lead-out leads 55, 57 are each bent near one end portion by about 90° in the horizontal direction in FIG. 26 toward directions to face each other, and each bent at one end portion by about 90° downward to form connecting portions 55a, 57a to be parallel to the electrode pins 71, 81. Further, the lead-out leads 56, 58 are each bent at one end portion by about 90° downward in FIG. 26 to form connecting portions 56a, 58a to be parallel to the electrode pins 72, 82.

Then, as shown in FIG. 26, the outer peripheral surfaces of projecting portions of the electrode pins 71, 72, 81, 82 projecting from the mounting base through the through-holes 12d of the holder with spring properties 12 and the outer peripheral surfaces of the connecting portions 55a, 56a, 57a, 58a being portions, each of which is made by bending one end portion of the lead-out lead 55 to 58, into parallel to the electrode pins, are brought into abutment with each other along their center axes, and the contacted portions are connected by laser welding. This makes it possible to further surely connect the electrodes to the lead-out leads.

Since the spacer cannot be fitted in advance because of the laser welding work in this embodiment, one half part 41 and an other half part 42 of the dual-partitioned spacer are arranged such that their cut surfaces are fitted from directions indicated by arrows in FIG. 27 and brought into contact with each other after laser welding, and held by spacer holding pieces 12a, 12a of the holder with spring properties 12. The connected portions between the electrode pins 71, 72, 81, 82 and the lead-out leads 55 to 58 are made to escape into an escape hole formed by half parts 41 a, 42a of the escape hole.

On the spacer composed of the one half part 41 and the other half part 42 of the spacer, a pressing member 60 is laid as shown in FIG. 28. In a surface of the pressing member 60 on the side in contact t with the spacer, a plurality of grooves 60b similar to the grooves 4b of the spacer 4 in the above-described embodiment are formed in each of directions perpendicular to each other so that the lead-out leads 55 to 58 are introduced in the grooves 60b and guided.

Then by bending the locking piece parts 12b₁ of the pair of pressing member locking pieces 12b, 12b of the holder with spring properties 12 inward by about 90° , the pressing member 60 is positioned in the radial direction and pressed against the one half part 41 and the other half part 42 of the spacer. Also into the grooves 60b of the pressing member 60, a ceramic adhesive, a glass adhesive or the like is filled to firmly fix them.

It is preferable also in this embodiment to process the connecting portions of the lead-out leads and the electrode pins to be flat to each other or to form one of them into a recessed cylindrical surface to thereby increase the contact area. However, even if they are not processed but are in thin cylindrical shapes as they are, they can be sufficiently connected. Further, the connecting method is not limited to the laser welding, but measures of electric resistance welding, bonding using a conductive adhesive, swaging or the like may be employed.

### Method of Manufacturing Porous Ceramic Part

A method of manufacturing the cap 3, the spacer 4, the pressing member 6 and the like which are cover members made of porous ceramics will be briefly described here.

First, a ceramic powder material and required additives are prepared, and all of them are uniformly mixed. In this case, as the ceramic powder material, powder of a general ceramic material such as alumina powder, zirconia powder or the like with a powder diameter of about 0.3 µm is used. As the additives, appropriate amounts of an auxiliary agent, a binder and pure water are used. Note that polyacrylate or the like can be used as the auxiliary agent, and acryl, PVA (polyvinyl alcohol), PEO (polyethyleneoxide) or the like can be used as the binder.

Thereafter, a spray drier or the like is used to produce a granulated material with an average grain diameter of 60 to 120 µ m. Then, the granulated material is pressed at a predetermined primary pressure and subjected to primary molding into a round pipe shape, for example, by injection molding. Thereafter, the primary molded body is subjected to primary baking (pre-baking) at a predetermined primary heating temperature to form a molded green compact. The primary heating temperature is selected from the range of 900 to 1200°C.

Then, the molded green compact is crushed and then classified into the grain diameters of 0.1 to 1.0 mm, and the green compact material is pressed at a predetermined secondary pressure and subjected to secondary molding into desired parts shape. The secondary molded body is subjected to secondary baking (main baking) at a predetermined secondary heating temperature. In this case, a temperature suitable for the ceramic powder material for use in a range of 1200 to 1600°C is appropriately set as the secondary heating temperature. In this manner, the parts such as the cap 3 and so on made of porous ceramics can be manufactured.

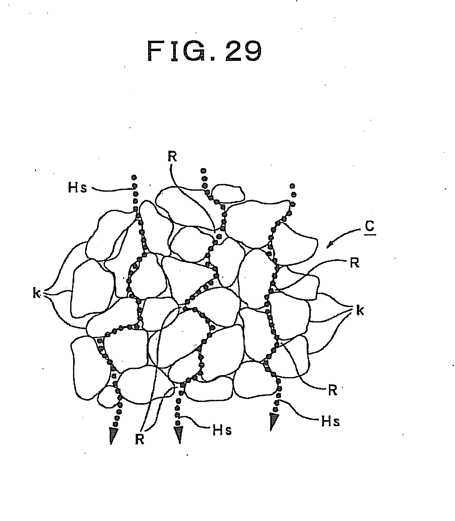

The porous ceramics are constituted by bonding of grains k-- of the green compact material as shown in FIG. 29 and pores R - - - are formed by spaces around the contact surface of the grain boundary, and therefore a predetermined gas permeability can be ensured by the gas passages along the pores R - - -. In this case, the width of the pores R - - - is less than 500 µ m, and invasion of moisture into the pores R - - - and invasion of unnecessary foreign substances other than gas is inhibited. The gas passages are shown by dotted arrows Hs - - - in FIG. 29.

As described above, for the porous ceramics C, the ceramic powder material and one additive or two or more additives are prepared to form a green compact material Po having a predetermined grain diameter, and the green compact material Po is subjected to primary molding at a predetermined primary pressure Ff and then subjected to primary baking at a predetermined primary heating temperature Tf to obtain a green compact material Pp having a size of grains k- - - raging from 0.1 to 1.0 mm.

The green compact material Pp is subjected to secondary molding at a predetermined secondary pressure Fs and then subjected to secondary baking at a predetermined secondary heating temperature Ts to sufficiently ensure both of the desired gas permeability and a desired mechanical strength (bend strength) when it is used in the gas sensor. Especially by selecting the size of the grains in the green compact material Po in the range of 60 to 120 µ m, optimal porous ceramics C which can sufficiently achieve those effects can be obtained.

### Conclusion and Applicable Range

Since the gas sensor according to the invention is configured such that the lead-out leads 51 to 53 or 55 to 58 extending in the direction parallel to the rear surface of the mounting base and the rear surface of the holder 1 are provided to connect with the electrode pins 71, 72 of the gas-sensitive element unit 7 and the electrode pins 81, 82 of the compensating element unit 8 projecting to the rear surface side of the mounting base 2 as described regarding the embodiments, the wiring work with a detection circuit board becomes easy, and since a large space behind the sensor is not required, the flexibility of freedom when the gas sensor is installed in a device is increased, and space saving can be achieved.

Further, the lead-out leads may be arranged on the same plane as in the above-described embodiments and may be made different in length to necessary. By connecting one of electrode pins in each pair of electrode pins of the gas-sensitive element unit and the compensating element unit to a common lead-out lead, the number of lead-out leads can be reduced to three. Further, if the lead-out leads are formed of a solderable metal material such as nickel or copper-nickel alloy or the like, the wiring can be made easier.

The above embodiments have described the examples in which the invention is applied to the catalytic combustion type gas sensor having a compensating element provided therein, but the invention is, of course, applicable to a catalytic combustion type gas sensor having a compensating element as a separated body and only the gas-sensitive element unit embedded therein. Further, the invention is also applicable to gas sensors including various gas-sensitive elements such as the above-described semiconductor type gas sensor, a solid electrolyte gas sensor and so on.

The number of electrode pins is not limited to four, but the invention is applicable to a gas sensor having a plurality of electrode pins such as two, three, five, or six, and the pin base and the pin stay are not essential but any of them may be omitted, or each electrode pin may directly pass through the mounting base to be held therein.

The portions near the connected portions between the electrode pins 71, 72, 81, 82 and the lead-out leads 51 to 53 are held and fixed in the mounting base and the holder board 11 by the holder with spring properties 12, the spacer 4, and the pressing member 6 in the above embodiments, but their shapes and materials may be variously changed.

Further, the mounting base, the spacer, and the pressing member can also be formed in a shape other than the circular plate shape (an elliptical plate shape, a square plate shape, a polygonal plate shape, a block shape or the like), and may be formed of a heat resistant synthetic resin. The holder with spring properties may be made in a shape adapted thereto.

Further, part or all of the holder with spring properties, the spacer, and the pressing member may be omitted, and the portions near the connected portions between the electrode pins and the lead-out leads may be fixed with a heat resistant adhesive, or covered with a cover and fixed by a heat resistant filler filled into the inside of the cover.

### INDUSTRIAL APPLICABILITY

The gas sensor according to the invention is widely applicable in devices detecting gas leakage or generation of toxic gas in apparatuses and systems using various kinds of flammable gases or in rooms where they are installed, and so on. Especially, the invention is similarly applicable also to detection of generation of toxic gas such as CO due to incomplete combustion, detection of hydrogen leakage in each partition inside a fuel cell vehicle whose rapid practical application in future is expected, a hydrogen gas sensor in a fuel cell system and the like used as an auxiliary power source for industrial use or for home use, or a gas sensor detecting non-flammable gas such as CO₂ or the like.

## Claims

1. A gas sensor, comprising:
a gas-sensitive element; a plurality of electrode pins each connected to said gas-sensitive element at one end portion and supporting said gas-sensitive element;
a mounting base made of an insulating material supporting said plurality of electrode pins each passing through from one surface to another surface; and
a gas-permeable cover member firmly fixed to said mounting base to cover a region on the one surface side of said mounting base including said gas-sensitive element,
wherein lead-out leads extending in a direction parallel to the another surface of said mounting base are provided respectively to connect with portions of said plurality of electrode pins projecting to the another surface side of said mounting base.

2. The gas sensor according to claim 1,
wherein a spacer through which said each electrode pin passes and which supports said each lead-out lead and a pressing member covering substantially the entire surface of said spacer on the side supporting said each lead-out lead are provided on the side of said mounting base where said each electrode pin projects, and both of said pressing member and said spacer are made of an insulating material.

3. A gas sensor, comprising:
a gas-sensitive element unit in which a plurality of electrode pins electrically connected to a gas-sensitive element and supporting said gas-sensitive element pass through a pin stay made of an insulating material and are held in parallel;
a mounting base holding said pin stay fitting therein;
a gas-permeable cover member firmly fixed to one surface side of said mounting base to cover said gas-sensitive element side of said gas-sensitive element unit; and
a holder having an opening from which said cover member projects, and fixing and holding said mounting base,
wherein lead-out leads extending in a direction parallel to another surface of said mounting base are provided respectively to connect with said electrode pins of said gas-sensitive element unit projecting to the another surface side of said mounting base.

4. A gas sensor, comprising:
a gas-sensitive element unit in which a pair of electrode pins electrically connected to both terminals of a gas-sensitive element and supporting said gas-sensitive element pass through a first pin stay made of a heat resistant insulating material and are held in parallel;
a compensating element unit in which a pair of electrode pins electrically connected to both terminals of a compensating element and supporting said compensating element pass through a second pin stay made of a heat resistant insulating material and are held in parallel;
a mounting base holding said first pin stay and said second pin stay fitting therein with said gas-sensitive element unit and said compensating element unit facing each other;
a gas-permeable cover member firmly fixed to one surface side of said mounting base to cover said gas-sensitive element side of said gas-sensitive element unit and said compensating element side of said compensating element unit; and
a holder having an opening from which said cover member projects, and fixing and holding said mounting base,
wherein lead-out leads extending in a direction parallel to another surface of said mounting base are provided respectively to connect with said electrode pins of said gas-sensitive element unit and said electrode pins of said compensating element unit projecting to the another surface side of said mounting base.

5. The gas sensor according to any one of claims 1 to 4,
wherein a tip end face of said electrode pin and a flattened portion of said lead-out lead are in contact with each other, and contacted portions of said electrode pin and said lead-out lead are connected by laser welding.

6. The gas sensor according to any one of claims 1 to 4,
wherein an outer peripheral surface of a portion of said electrode pin projecting from said mounting base and an outer peripheral surface of a portion of said lead-out lead in parallel to said electrode pin made by bending at one end portion are in contact with each other along respective center axes, and contacted portions of said electrode pin and said lead-out lead are connected by laser welding.

7. The gas sensor according to claim 3 or 4,
wherein a spacer through which said each electrode pin passes and which supports said each lead-out lead and a pressing member covering substantially the entire surface of said spacer on the side supporting said each lead-out lead are provided on the side of said holder where said each electrode pin projects, and both of said pressing member and said spacer are made of an insulating material.

8. The sensor according to claim 7,
wherein at said holder, a plurality of spacer holding pieces holding an outer peripheral surface of said spacer and a plurality of pressing member locking pieces locking said pressing member by pressing said pressing member toward said spacer side are integrally formed of a metal plate with spring properties.

9. The gas sensor according to any one of claims 2, 7 and 8,
wherein a groove guiding said each lead-out lead is formed in a surface of said spacer on the side supporting said each lead-out lead.

10. The gas sensor according to claim 9,
wherein a plurality of said grooves are formed in each of directions perpendicular to each other, and said each lead-out lead connected to said each electrode pin has a bent portion at least one place along said groove.

11. The gas sensor according to any one of claims 2, 7 and 8,
wherein a groove guiding said each lead-out lead is formed in a surface of said pressing member on a side in contact with said spacer.

12. The gas sensor according to claim 11,
wherein a plurality of said grooves are formed in each of directions perpendicular to each other, and said each lead-out lead connected to said each electrode pin has a bent portion at least one place along said groove.

13. The gas sensor according to any one of claims 2 and 7 to 12,
wherein a plurality of grooves or projections and depressions for increasing the surface area are formed on a surface of said pressing member on a side not in contact with said spacer.

14. The gas sensor according to any one of claims 2 and 7 to 13,
wherein at least one of said spacer and said pressing member is made of ceramics or porous ceramics.

15. The gas sensor according to any one of claims 1 to 14,
wherein said cover member is made of porous ceramics.

16. The gas sensor according to any one of claims 1 to 15,
wherein said mounting base is made of ceramics or porous ceramics.

17. The gas sensor according to claim 3,
wherein said mounting base has a fitting slot for fitting said pin stay therein, and said fitting slot is in an opening shape equal to or slightly larger than the outer peripheral shape of said pin stay from the another surface side of said mounting base to a middle in the thickness direction, and the opening shape is reduced in size from the middle to the one surface side to form a stepped part, and
wherein a cutout part being an escape for said gas-sensitive element to pass through when said pin stay is fitted in said fitting slot is formed in said stepped part.

18. The gas sensor according to claim 3,
wherein said mounting base has a fitting slot for fitting said pin stay therein, and said fitting slot is in an opening shape equal to or slightly larger than the outer peripheral shape of said pin stay from the another surface side of said mounting base to a middle in the thickness direction, and the opening shape is reduced in size from the middle to the one surface side to form a stepped part, and
wherein a pressing spring pressing said pin stay fitted in said fitting slot against said stepped part by pressing said pin stay from a rear surface thereof is interposed between said holder and said mounting base.

19. The gas sensor according to claim 4,
wherein said mounting base has a pair of fitting slots for fitting said first pin stay and said second pin stay therein respectively, and each of said fitting slots is in an opening shape equal to or slightly larger than the outer peripheral shape of said first or second pin stay from the another surface side of said mounting base to a middle in the thickness direction, and the opening shape is reduced in size from the middle to the one surface side to form a stepped part, and cutout parts being escapes for said gas-sensitive element and said compensating element to pass through when said first pin stay and said second pin stay are fitted respectively in said pair of fitting slots are formed in said stepped parts.

20. The gas sensor according to claim 4,
wherein said mounting base has a pair of fitting slots for fitting said first pin stay and said second pin stay therein respectively, and each of said fitting slots is in an opening shape equal to or slightly larger than the outer peripheral shape of said first or second pin stay from the another surface side of said mounting base to a middle in the thickness direction, and the opening shape is reduced in size from the middle to the one surface side to form a stepped part, and
wherein a pressing spring pressing said first pin stay and said second pin stay respectively fitted in said pair of fitting slots against said stepped parts by pressing said first pin stay and said second pin stay from rear surfaces thereof is interposed between said holder and said mounting base.

21. The gas sensor according to any one of claims 4, 19 and 20,
wherein a heat shielding plate for thermally shielding said gas-sensitive element and said compensating element in said cover member is provided at said mounting base.

22. The gas sensor according to claim 21,
wherein said heat shielding plate is made of ceramics or porous ceramics.
